(19) Europäisches Patentamt
European Patent Office
Office européen des brevets

(11) **EP 1 842 437 A1**

(12) **EUROPEAN PATENT APPLICATION**

(43) Date of publication:
**10.10.2007 Bulletin 2007/41**

(51) Int Cl.:
*A23L 3/3508* [(2006.01)]    *A61L 2/16* [(2006.01)]

(21) Application number: **06112286.7**

(22) Date of filing: **06.04.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: PURAC Biochem BV
**4200 AA  Gorinchem (NL)**

(72) Inventors:
• **Roelf, Otto**
  **4201 BC, Gorinchem (NL)**
• **Ramirez, Aldana, Mariel**
  **6702 AG, Wageningen (NL)**

(74) Representative: **Beetz, Tom**
**De Vries & Metman**
**Overschiestraat 180**
**1062 XK Amsterdam (NL)**

(54) **Antimicrobial preparations**

(57)    The invention pertains to an antimicrobial preparation directed against Gram-negative bacteria comprising at least two amino acids chosen from at least one of group I, group II, and group III, wherein group I is the group of neutral amino acids consisting of glycine and alanine, group II is the group of hydroxy-containing amino acids consisting of serine and threonine, and group III is the group of basic amino acids consisting of arginine, lysine, and ornithine, or directed against Listeria *monocytogenes* comprising the combination DL-threonine and glycine.
The invention further relates to a method for preserving food against bacteria comprising adding to the food said antimicrobial preparations.

**Description**

The invention relates to an antimicrobial preparation and to a process of preserving food. In particular, it relates to a process of preserving food with a combination of two or more amino acids.

The morbidity and mortality associated with the consumption of food contaminated by infectious and toxin-producing microorganisms should not be underestimated. It is for this reason that the microbial quality and safety of food is a cause of constant concern for food processors, consumers and government agencies. Furthermore the spoilage and putrefaction of a foodstuff by microorganisms can compromise its nutritional value and can lead to substantial economic losses. Microbial spoilage of food results from the uncontrolled proliferation or activities of microorganisms. To prevent this preservation technologies have been developed which ensure the quality and microbiological safety of foods. These technologies are divers and include (i) procedures that prevent access of microorganisms to foods; (ii) procedures that inactivate microorganisms; and (iii) procedures that prevent or slow down the growth of microorganisms. Changes in consumer demands and food legislation actuate food technologists to constantly modify and improve existing food-processing technologies and invent and develop new ones. The trend is to produce foods, which not only look attractive, fresher and more natural but which combine this with the convenience of use such as a long shelf-life and ease of preparation. Furthermore the customer also sets high standards with respect to flavor, texture, appearance and safety. To accomplish all this presents a major challenge for food technologists. With respect to the slowing down or prevention of growth of microorganisms in foods new applications for new and more natural chemical antimicrobials are steadily being made. These new chemical preservatives, however, should satisfy a number of requirements:(i) they should have effective bactericidal or bacteriostatic activity against a wide range of different spoilage organisms and food-borne pathogens; (ii) they should not affect the appearance, taste, flavor or texture of the food; and (iii) they should not be toxic for the consumer. It is known that certain amino acids posses bactericidal or bacteriostatic properties (e.g. Shive, W.; C.G.Skinner (1963) Amino acid analogues. In: Metabolic inhibitors. A comprehensive treatise (Hochster,R.M.; J.H.Quastel eds). Academic Press. New York. Volume I, pp 1 - 73).

Furthermore, many amino acids occur naturally in foods and are generally regarded as save and approved for use in food products. It is exactly for these reasons that some amino acids notably glycine have found commercial application as preservatives.

Processes for preserving foodstuffs with serine were disclosed in US 2,711,976 and US 6,602,532. In US 3,615,703 the flavor of foodstuffs or beverages comprising a fruit or fruit juice, vegetable or vegetable juice, or beer is preserved by hermetically sealing the foodstuff or beverage in a container with either lysine, ornithine, histidine, or a salt thereof. GB 1,510,942 discloses a process for the preservation of foodstuffs, particularly those containing 10-60% sugar, e.g. jellies, jams and custards which are protected against putrefaction by incorporating maltose and glycine in the foodstuffs. Glycine is suitably present in an amount of 0.3 to 2%. Although glycine is now widely used as a commercial preservative it is also recognized that this compound is not a strong preservative and relatively high concentrations are needed to bring about bacterial growth inhibition. These high concentrations, however, create a new set of problems. It is known that glycine, alanine, serine, and threonine all possess, to a different degree a sweet taste, lysine and ornithine both possess bitter and sweet notes whilst arginine is intensely bitter. The impact of amino acids on taste limits the application of these compounds as food preservatives.

In order for glycine to be used more effectively as a food preservative, there is therefore a demand for other substances that might be used in combination with glycine and help to increase its antimicrobial effect. Lee et al. have examined the antimicrobial effect of glycine in combination with hexametaphosphate, EDTA, cholic acid, and glycerol monocaprate on a number of Gram-positive and negative bacterial species (Lee,J.K.; K.Tatsuguchi; M.Tsutsumi; T.Watanabe, J.Food Hyg.Soc. Japan 26: 279 - 284 (1985)). In WO 01/56408 alanine or glycine was used together with 1,5-D-anhydrofructose to obtain a food-keeping agent which contain highly safe antibacterial substances and thus can improve the keeping qualities of foods without exerting any undesirable effects on the taste or flavor of the foods.

Although processes were disclosed using antimicrobial mixtures consisting of a single amino acid with one or more other non-amino acid compounds (see above), much less is known about the antimicrobial properties of mixtures of amino acids.

JP 2000-224976 discloses a process for the inhibition of microorganisms such as the lactic acid bacterium *Enterococcus faecalis* within a pH region of ≥6.5 of the food, and further hardly damaging the quality of the food using a mixture of calcium lactate and glycine and a salt of some other organic acid. Although in the same application it was also disclosed that part of the glycine can be substituted with alanine, the data show that alanine and mixtures of alanine and glycine are less effective inhibitors of growth of the Gram-positive bacterium *Enterococcus faecalis* than glycine alone. That alanine is able to counteract the inhibitory effect of glycine has not only been shown in case of the closely related Gram-positive bacteria *Enterococcus hirae* and *Lactococcus lactis* but also in the Gram-negative *Escherichia coli* (Snell, E.E.; B.M. Guirard (1943) Proc.Natl.Acad.Sci.USA 29: 66 - 73, Hishinuma, F.; K. Izaki; H. Takahashi (1969) Effects of glycine and D-amino acids on the growth of various micro organisms Agr.Biol.Chem. 33: 1577 - 1586). That one amino acid can cancel out the inhibition exerted by another amino acid is well known and has been observed in a number of Gram-positive and Gram-negative bacterial species e.g. in the Gram-positive bacteria: *Bacillus anthracis* and *Listeria monocytogenes,* and in the Gram-negative bacterium *Escherichia coli* (Gladstone,G.P. (1939) Brit.J.Exp.Pathol. 20: 189 - 200; Friedman,M.E.; W.G.Roessler (1961) J.Bacteriol. 82: 528 - 533; de Felice et al. (1979) Microbiol. Rev. 43: 42 - 58).

To our surprise it was now found that certain combinations of amino acids exert a synergy in inhibition of Gram-negative bacteria and in *Listeria monocytogenes,* and that this type of interaction between structurally different amino acids is common.

To this end the invention pertains to an antimicrobial preparation directed against Gram-negative bacteria comprising at least two amino acids chosen from at least one of group I, group II, and group III, wherein group I is the group of neutral amino acids consisting of glycine and alanine, group II is the group of hydroxy-containing amino acids consisting of serine and threonine, and group III is the group of basic amino acids consisting of arginine, lysine, and ornithine, or directed against *Listeria monocytogenes* comprising the combination DL-threonine and glycine.

Thus the present antimicrobial preparation contains for instance two amino acids from group I, i.e. the combination of glycine and alanine, two amino acids from group II, i.e. the combination of serine and threonine, or two amino acids from group III, i.e. the combination of arginine and lysine, or arginine and ornithine, or lysine and ornithine, or a combination of one amino acid from group I and one amino acid from group II, such as glycine or alanine with serine or threonine; or a combination of one amino acid from group I and one amino acid from group III such as the combination of glycine or alanine with arginine, lysine, or ornithine, or a combination of one amino acid from group II and one amino acid from group III, such as the combination of serine or threonine with arginine, lysine, or ornithine. A particularly useful example of such combination is glycine (group I) and threonine (group II) for inhibiting the growth of both Gram-negative bacteria and *Listeria monocytogenes,* and the combination of glycine and alanine for inhibiting the growth of *Escherichia coli* 0157:H7 and *Salmonella* species.

It is stressed that any one of these combinations of two amino acids can be further combined with a third, or even fourth, etc. amino acid. It was found that these above combinations have an antibacterial effect that is greater than could be expected on the basis of the amino acids in isolation from other amino acids, i.e. show a synergistic effect.

The combination of amino acids preferably comprises D-amino acids. Most preferably, the antimicrobial preparation contains DL-amino acids.

In another aspect, the invention pertains to a method for preserving food against bacteria comprising adding to the food any one of the above-mentioned antimicrobial preparations.

The method according to the invention is particularly useful for protecting the food against at least one of *Salmonella enterica, Escherichia coli* 0157:H7, and *Listeria monocytogenes.*

Examples of such food products are meat products (cured and/or uncured, fresh and/or cooked), salads and other vegetable products, drinks and dairy products, semi-processed foods, convenient foods as e.g. ready-to-eat meals and dried food products. The method is of particular interest since some fresh meat products are used for direct consumption (e.g. filet américain, steak tartar, sushi, or carpaccio) without any heat treatment or with heat treatment insufficient to kill bacteria. Other meat products are consumed after application of only partial heat treatment, intentionally applied as e.g. for medium cooked steak or unintentionally applied due to improper preparation or improper handling of the food products.

The antimicrobial preparations of the invention can further be applied in other applications as well such as controlling the intestinal flora by inhibiting Gram-negative bacteria such as *Salmonella* and *Escherichia* to provide a selective advantage to Gram-positive bacteria such as Lactobacilli, for instance by administration together with probiotic bacteria.

The following cultures were used in a study: *Escherichia coli* serotype 0157:H7 (ATCC 700728), *Listeria monocytogenes* (ATCC 7644), *Salmonella enterica* (ATCC 13311). All cultures were transferred daily in screw-capped tubes (100 x 16 mm) containing 10 ml brain heart infusion broth (Oxoid, Basingstoke, UK). Cultures were incubated at 30° C without agitation. Brain heart infusion broth was prepared with increasing amounts of two different amino acids. The concentration ranges for the different amino acids were as follows: DL-threonine, glycine, DL-alanine, DL-ornithine from 0 to 450 mM in 10 50 mM steps, DL-serine from 0 to 225 mM in 10 25 mM steps, DL-lysine, DL-arginine from 0 to 270 mM in 10 30 mM steps. For each binary mixture this resulted in 90 different media. The pH of the media was adjusted to 6.1 - 6.2. Media were prepared in 10 ml quantities and sterilised by filtration (Sartorius cellulose nitrate membranes 0.45 $\mu$m pore diameter). 300 $\mu$l of each medium was transferred to a panel of a sterile Bioscreen honeycomb 100 well plate. Well plates were inoculated with 5 $\mu$l of a culture that was grown overnight in brain heart infusion broth using a sterile Hamilton 5 $\mu$l repeating dispenser (Hamilton, Bonaduz, Switzerland). Growth rates were determined with a Bioscreen C (Labsystems, Helsinki, Finland) that kinetically measures the development of turbidity by vertical photometry. The plates were incubated for 16 - 24 hours at 37° C, the optical density of the cultures was measured every 30 minutes at 420 - 580 nm using a wide band filter. The Bioscreen measures at set time intervals the optical density of the cultures. From these data the Bioscreen calculates maximum specific growth rates.

The purpose of further data processing is to ascertain whether two amino acids act independently of each other or whether they stimulate each other in their inhibitory action (synergy) or cancel out each other inhibitory effect (antagonism). When a certain compound has no effect on an organism the specific growth rate of this organism ($\mu$) can be expressed as a function (f) of the growth limiting substrate concentration (s) by for example the Monod equation, which reads: $\mu = \mu_{max} \cdot s / (K_s + s)$, where $\mu_{max}$ represents the maximum specific growth rate, s the standing concentration of the growth limiting substrate in the medium and $K_s$ the substrate concentration where $\mu = 0.5 \mu_{max}$. However, when the presence of an inhibitor P affects cell growth the function f for $\mu$ must be modified i.e. $\mu = f(s,p)$, where p represents the concentration of inhibitor P. Numerous studies of growth inhibition kinetics of bacteria have shown that many inhibitors behave as non-competitive inhibitors. This implies that only the maximum specific growth rate ($\mu_{max}$) value and not the affinity ($K_s$) is affected. Therefore the specific growth rate in the presence of inhibitor can be written as: $\mu = \mu_i \cdot s / (K_s + s)$, where $\mu_i$ is the maximal specific growth rate in the presence of a inhibitor P. The relationship between $\mu_i$ and $\mu_{max}$ and the concentration of the inhibitor P was describes using the Logistic Dose Response equation, which reads: $\mu_i / \mu_{max} = 1 / (1 + (p / p_{0.5})^b)$ (Jungbauer, A. (2001). The logistic dose response function: a robust fitting function for transition phenomena in life sciences. J.Clinical Ligand Assay 24: 270 - 274). In this equation p represents the concentration of inhibitor P and $p_{0.5}$ the concentration of P where $\mu_i = 0.5 \mu_{max}$ ; $\mu_{max}$ is the maximum specific growth rate that is the specific growth rate in the absence of inhibitor P, b is a dimensionless quantity, which determines the relationship between $\mu_i$ and p. Combining the Monod and Logistic Dose Response equation it can be written as: $\mu = \mu_{max} (s / K_s + s) / (1 + (p / p_{0.5})^b)$. In batch culture where s is usually many times higher than $K_s$ this equation reduces to $\mu = \mu_{max} / (1 + (p / p_{0.5})^b)$.

When comparing different organisms grown under the same conditions, or the same organism grown under different conditions, it is more meaningful to use relative growth rate, rather than absolute growth rates as standards of comparison. Relative growth rate (O) is the ratio of growth rate (p) to maximum growth rate ($\mu_{max}$) i.e. $O = \mu / \mu_{max}$. It can be seen that while $\mu$ and $\mu_{max}$ have the dimensions of $(time)^{-1}$, their ratio O is dimensionless, i.e. a pure number. Similarly we can define the relative inhibitor concentration $\varepsilon$ as $p / p_{0.5}$. The reduced Monod and Logistic Dose Response equation can now be written as: $O = 1 / (1 + \varepsilon^b)$. For two inhibitors X and Y e.g. the following two expressions for O can be defined:

$$O_x = 1 / (1 + \varepsilon^{b1}) \text{ and } O_y = 1 / (1 + \varepsilon^{b2}).$$

$O_x$ and $O_y$ can be experimentally evaluated by examining the inhibitory effects of either X or Y on the growth rate of the target organism. Knowing the evaluated functions for $O_x$ and $O_y$ the theoretical independent effect is defined as: $O_x.O_y$. The experimentally observed effect of combinations of X and Y on the relative growth rate is defined as $O_{xy}$. The hypothesis that X and Y act independently of each other on a certain organism is mathematically translated to $O_{xy} / O_x.O_y = 1$. Rejection of this hypothesis implies that the combined effect of X and Y is not an additive effect but either synergistic or antagonistic. In case the inhibitors X and Y act synergistically upon the target organism $O_{xy} / O_x.O_y < 1$ (but > 0). In those cases that the combined effect of inhibitors X and Y is antagonistic $O_{xy} / O_x.O_y > 1$. Synergy, independent effect, and antagonism can be visualized in a plot of $O_{xy}$ versus $O_x.O_y$.

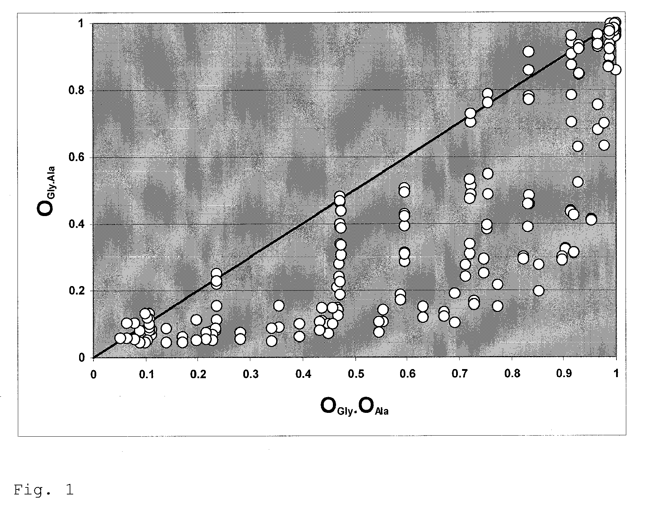

This is exemplified in Figs. 1-3. In Fig. 1 a plot is given of $O_{Gly.Ala}$ (experimentally observed relative growth rate) versus $O_{Gly}.O_{Ala}$ (predicted relative growth rate) for *Escherichia coli* 0157:H7 (ATCC 700728) showing the synergy in inhibition between Glycine and DL-alanine. The solid line in this graph represents the line where the experimentally observed relative growth rate ($O_{Gly.Ala}$) equals the predicted relative growth rate ($O_{Gly}.O_{Ala}$) and where the Gly and Ala act as independent inhibitors.

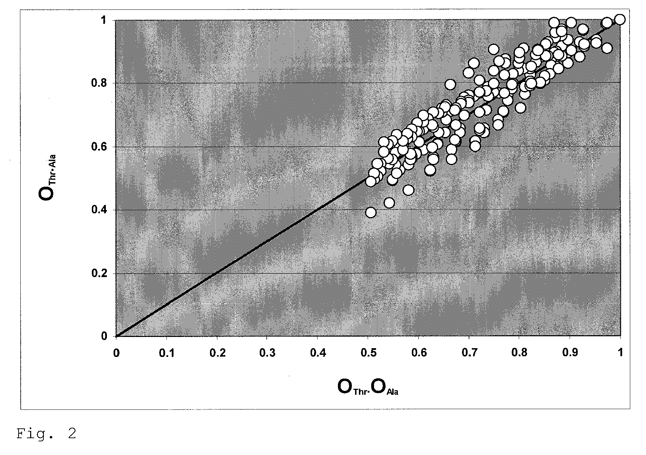

In Fig. 2 a plot is given of OThr.Ala (experimentally observed relative growth rate) versus OThr.$O_{Ala}$ (predicted relative growth rate) for *Listeria monocytogenes* (ATCC 7644) showing the independent effect of DL-Threonine and DL-alanine. The solid line in this graph represents the line where the experimentally observed relative growth rate ($O_{Thr.Ala}$) equals the predicted relative growth rate ($O_{Thr}.O_{Ala}$) and where threonine and alanine act as independent inhibitors.

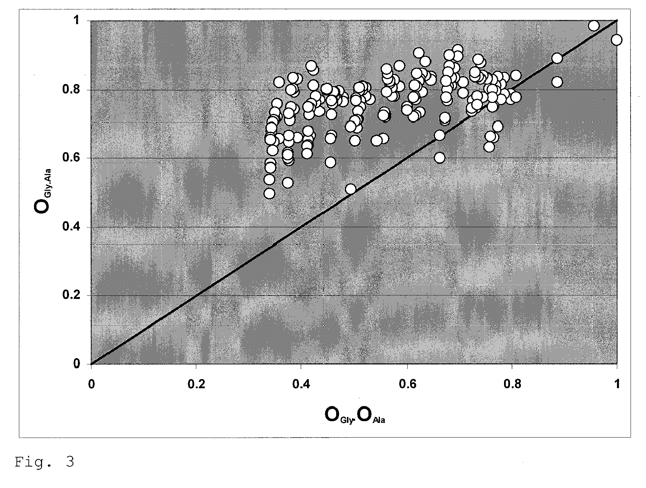

In Fig. 3 a plot is given of $O_{Gly.Ala}$ (experimentally observed relative growth rate) versus $O_{Gly}.O_{Ala}$ (predicted relative growth rate) for *Listeria monocytogenes* (ATCC 7644) showing the antagonistic effect of Glycine and DL-alanine. The solid line in this graph represents the line where the experimentally observed relative growth rate ($O_{Gly.Ala}$) equals the predicted relative growth rate ($O_{Gly}.O_{Ala}$) and where Glycine and alanine act as independent inhibitors.

The following are specific non-limitative examples of suitable amino acid combinations, all of which show a synergistic effect against bacteria.

Two amino acids chosen from group I

The combination of DL-alanine and glycine is particularly effective against *Salmonella enterica* and *Escherichia coli* O157:H7 (synergism)

Two amino acids chosen from group II

The combination of DL-serine and DL-threonine is particularly effective against *Escherichia coli* 0157:H7 and *Staphylococcus aureus* (synergism).

Two amino acid chosen from group III

Combinations of DL-arginine and DL-lysine, DL-arginine and DL-ornithine, DL-lysine and DL-ornithine are particularly effective against *Salmonella enterica, Escherichia coli* 0157:H7 and *Staphylococcus aureus.*

Two amino acids chosen from group I and group II

The combination of DL-serine and glycine is particularly effective against *Salmonella enterica, Escherichia coli* 0157:H7, and *Staphylococcus aureus;* the combination of DL-serine and DL-alanine is particularly effective against *Escherichia coli* 0157:H7 and *Salmonella enterica and* the combination of DL-threonine and glycine which is particularly effective against *Escherichia coli* 0157:H7, *Staphylococcus aureus* and *Listeria monocytogenes.*

Two amino acids chosen from group I and group III

The combination of DL-arginine and glycine is particularly effective against *Salmonella enterica, Escherichia coli* 0157:H7 and *Staphylococcus aureus;* DL-Arg/DL-Ala, particularly effective against *Salmonella enterica, Escherichia coli* 0157:H7; the combination of DL-lysine and glycine is particularly effective against *Salmonella enterica* and *Escherichia coli* 0157:H7; the combination of DL-lysine and DL-alanine is particularly effective against *Salmonella enterica, Escherichia coli* 0157:H7 and *Staphylococcus aureus;* and the combination of DL-ornithine and glycine which is particularly effective against *Staphylococcus aureus.*

Two amino acids chosen from group II and group III

The combination of DL-arginine and DL-serine is particularly effective against *Escherichia coli* 0157:H7 and *Staphylococcus aureus;* the combination of DL-arginine and DL-threonine is particularly effective against *Escherichia coli* 0157:H7; the combination of DL-lysine and DL-serine is particularly effective against *Escherichia coli* 0157:H7, *Salmonella enterica* and *Staphylococcus aureus;* the combination of DL-lysine and DL-threonine is particularly effective against *Escherichia coli* 0157:H7, *Salmonella enterica* and *Staphylococcus aureus;* the combination of DL-ornithine and DL-serine is particularly effective against *Salmonella enterica* and the combination of DL-ornithine and DL-threonine is particularly effective against *Escherichia coli* 0157:H7 and *Staphylococcus aureus.*

Competition between *Escherichia coli* 0157:H7 (ATCC 700728) and *Lactobacillus plantarum* (DSM 20174) (mixed culture A), and between *Salmonella enterica* (ATCC 13311) and *Lactobacillus plantarum* (DSM 20174) (mixed culture B) was studied in broth cultures. *Escherichia coli, Salmonella enterica*, and *Lactobacillus plantarum* were transferred daily in screw-capped tubes (100 x 16 mm) containing 10 ml brain heart infusion broth (Oxoid, Basingstoke, UK). Cultures were incubated at 30° C without agitation. 500 $\mu$l of an overnight culture of *Escherichia coli* and 5 $\mu$l of a culture of *Lactobacillus plantarum* were transferred to screw capped tubes containing 10 ml of freshly prepared brain heart infusion broth or to 10 ml of brain heart infusion broth containing 200 mM of DL-alanine and 200 mM of glycine or 400 mM of DL-alanine and 400 mM of glycine (mixed culture A, first transfer). 500 $\mu$l of an overnight culture of *Salmonella enterica* and 5 $\mu$l of a culture of *Lactobacillus plantarum* were transferred to screw capped tubes containing 10 ml of freshly prepared brain heart infusion broth or to 10 ml of brain heart infusion broth containing 200 mM of DL-alanine and 200 mM of glycine or 400 mM of DL-alanine and 400 mM of glycine (mixed culture B, first transfer). Both mixed cultures were incubated at 30° C. After 24 hours the cultures were transferred to fresh media (second transfer) and also plated on Violet Red Bile agar (Oxoid, Basingstoke, CM0485) and MRS agar (Oxoid Basingstoke, CM0361). The second transfer was incubated for 24 hours at 30° C and subsequently plated on Violet Red Bile agar and MRS agar. The results of this analysis, which are summarized in Table 1 demonstrate the competitive advantage of *Lactobacillus plantarum* in a mixed culture with either *Escherichia coli* and *Salmonella enterica* in a medium containing DL-alanine and glycine.

Table 1. Colony forming units (cfu) per ml broth in the first and second transfer.

| | Mixed culture A cfu / ml in first transfer | | Mixed culture BI cfu / ml in first transfer | |
|---|---|---|---|---|
| Additions to BHI | *E.coli* 0157:H7 ATCC 700728 | *Lb. plantarum* DSM 20174 | *Salmonella* ATCC 13311 | *Lb. plantarum* DSM 20174 |

(continued)

| | Mixed culture A cfu / ml in first transfer | | Mixed culture BI cfu / ml in first transfer | |
|---|---|---|---|---|
| None | $390 . 10^6$ | $268 . 10^6$ | $60 . 10^6$ | $277 . 10^6$ |
| 200 mM Glycine 200 mM DL-Alanine | $25 . 10^6$ | $264 . 10^6$ | $15 . 10^6$ | $660 . 10^6$ |
| 400 mM Gly 400 mM DL-Alanine | 0 | $246 . 10^6$ | 0 | $322 . 10^6$ |

| | Mixed culture A cfu / ml in second transfer | | Mixed culture B cfu / ml in second transfer | |
|---|---|---|---|---|
| Additions to BHI | *E.coli* 0157:H7 ATCC 700728 | *Lb. plantarum* DSM 20174 | *Salmonella* ATCC 13311 | *Lb. plantarum* DSM 20174 |
| None | $291 . 10^6$ | $630 . 10^6$ | $40 . 10^6$ | $780 . 10^6$ |
| 200 mM Glycine 200 mM DL-Alanine | $16 . 10^6$ | $690 . 10^6$ | 0 | $750 . 10^6$ |
| 400 mM Glycine 400 mM DL-Alanine | 0 | $610 . 10^6$ | 0 | $940 . 10^6$ |

## Claims

1. An antimicrobial preparation directed against Gram-negative bacteria comprising at least two amino acids chosen from at least one of group I, group II, and group III, wherein group I is the group of neutral amino acids consisting of glycine and alanine, group II is the group of hydroxy-containing amino acids consisting of serine and threonine, and group III is the group of basic amino acids consisting of arginine, lysine, and ornithine, or directed against *Listeria* monocytogenes comprising the combination DL-threonine and glycine.

2. The antimicrobial preparation of claim 1 wherein at least one amino acid is from group I and at least one amino acid is from group II or III.

3. The antimicrobial preparation of claim 2 wherein one amino acid is glycine and one amino acid is from group II or III.

4. The antimicrobial preparation of claim 3 wherein one amino acid is threonine and one amino acid is glycine.

5. The antimicrobial preparation of any one of claims 1-4 wherein the amino acids other than glycine are DL-amino acids.

6. A method for preserving food against bacteria comprising adding to the food the antimicrobial preparation of any one of claims 1-5.

7. The method according to claim 6 wherein the food is protected against at least one of Salmonella, Escherichia coli and Listeria monocytogenes.

8. The method according to claim 6 wherein the food is protected against at least one of *Salmonella enterica, Escherichia coli* 0157:H7, and *Listeria monocytogenes.*

Fig. 1

Fig. 2

Fig. 3

European Patent
Office

**EUROPEAN SEARCH REPORT**

Application Number

EP 06 11 2286

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2001/033884 A1 (YAMADA KAZUKO ET AL) 25 October 2001 (2001-10-25) * figures 16,18 * * paragraphs [0014], [0015], [0040] - [0043], [0047], [0049], [0052] * | 1-3,5-8 | INV. A23L3/3508 A61L2/16 |
| A | * paragraph [0047] * | 4 | |
| X | "Preservation of paste type foods e.g. fish or artificial meat - by making the paste weakly alkaline then adding glycine and/or L-serine and alkali metal salt of organic acid" DERWENT, 1978, XP002186584 * the whole document * | 1-3,5-8 | |
| D,X | PATENT ABSTRACTS OF JAPAN vol. 2000, no. 11, 3 January 2001 (2001-01-03) & JP 2000 224976 A (SHINKOO GIJUTSU KAIHATSU CENTER:KK; KIMURA KAZUTAKA), 15 August 2000 (2000-08-15) | 1,5,6 | TECHNICAL FIELDS SEARCHED (IPC) |
| A | * abstract * | 2-4,7,8 | A23L A61L |
| X | US 2001/039264 A1 (ABE TAKASHI ET AL) 8 November 2001 (2001-11-08) * paragraphs [0006], [0008], [0015] * | 1-4 | |
| A | * paragraphs [0007], [0027] * | 5-8 | |
| X | US 2002/144946 A1 (DRAUZ KARLHEINZ ET AL) 10 October 2002 (2002-10-10) * paragraph [0013] * * table 1 * | 1-4 | |
| A | * paragraph [0012] * | 6-8 | |
| A | EP 1 629 724 A (PURAC BIOCHEM BV) 1 March 2006 (2006-03-01) * paragraphs [0011], [0017] * | 1-8 | |

-/--

The present search report has been drawn up for all claims

| Place of search | Date of completion of the search | Examiner |
|---|---|---|
| Munich | 22 May 2006 | Couzy, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
............................................................................
& : member of the same patent family, corresponding document

EPO FORM 1503 03.82 (P04C01)

**European Patent Office**

# EUROPEAN SEARCH REPORT

Application Number

EP 06 11 2286

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 4 461 777 A (MURASE ET AL) 24 July 1984 (1984-07-24) * column 1, lines 6-10 * * column 2, lines 5-10,30-40 * ----- | 1-8 | |

TECHNICAL FIELDS SEARCHED (IPC)

The present search report has been drawn up for all claims

| Place of search | Date of completion of the search | Examiner |
|---|---|---|
| Munich | 22 May 2006 | Couzy, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

EP 1 842 437 A1

**ANNEX TO THE EUROPEAN SEARCH REPORT
ON EUROPEAN PATENT APPLICATION NO.**

EP 06 11 2286

This annex lists the patent family members relating to the patent documents cited in the above-mentioned European search report.
The members are as contained in the European Patent Office EDP file on
The European Patent Office is in no way liable for these particulars which are merely given for the purpose of information.

22-05-2006

| Patent document cited in search report | | Publication date | Patent family member(s) | | Publication date |
|---|---|---|---|---|---|
| US 2001033884 | A1 | 25-10-2001 | NONE | | |
| JP 2000224976 | A | 15-08-2000 | NONE | | |
| US 2001039264 | A1 | 08-11-2001 | NONE | | |
| US 2002144946 | A1 | 10-10-2002 | NONE | | |
| EP 1629724 | A | 01-03-2006 | WO | 2006021587 A1 | 02-03-2006 |
| | | | WO | 2006021588 A1 | 02-03-2006 |
| | | | WO | 2006021589 A1 | 02-03-2006 |
| US 4461777 | A | 24-07-1984 | JP | 1614794 C | 15-08-1991 |
| | | | JP | 58031951 A | 24-02-1983 |
| | | | JP | 59016749 B | 17-04-1984 |
| | | | KR | 8801177 B1 | 02-07-1988 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2711976 A
- US 6602532 B
- US 3615703 A
- GB 1510942 A
- WO 0156408 A
- JP 2000224976 A

**Non-patent literature cited in the description**

- Amino acid analogues. **SHIVE,W. ; C.G.SKINNER.** Metabolic inhibitors. A comprehensive treatise. Academic Press, 1963, vol. I, 1-73
- **LEE,J.K. ; K.TATSUGUCHI ; M.TSUTSUMI ; T. WATANABE.** *J.Food Hyg.Soc. Japan,* 1985, vol. 26, 279-284
- **SNELL, E.E. ; B.M. GUIRARD.** *Proc.Natl.Acad.Sci. USA,* 1943, vol. 29, 66-73
- **HISHINUMA, F. ; K. IZAKI ; H. TAKAHASHI.** Effects of glycine and D-amino acids on the growth of various micro organisms. *Agr.Biol.Chem,* 1969, vol. 33, 1577-1586
- **GLADSTONE,G.P.** *Brit.J.Exp.Pathol,* 1939, vol. 20, 189-200
- **FRIEDMAN,M.E. ; W.G.ROESSLER.** *J.Bacteriol,* 1961, vol. 82, 528-533
- **DE FELICE et al.** *Microbiol. Rev,* 1979, vol. 43, 42-58
- **JUNGBAUER, A.** The logistic dose response function: a robust fitting function for transition phenomena in life sciences. *J.Clinical Ligand Assay,* 2001, vol. 24, 270-274